# EUROPEAN PATENT SPECIFICATION

(11) **EP 4 517 099 B1**
(45) Date of publication and mention of the grant of the patent: **15.10.2025**
(21) Application number: 24197304.9
(22) Date of filing: 29.08.2024
(51) Int. Cl.: F04D 29/02, C02F 1/467, F04D 29/42, A61L 2/03

(54) **PREVENTION OF MICROBIOLOGICAL GROWTH IN PROCESSING EQUIPMENT HAVING A ROTATING ELEMENT**
VERHINDERUNG VON MIKROBIOLOGISCHEM WACHSTUM IN VERARBEITUNGSAUSRÜSTUNG MIT EINEM ROTIERENDEN ELEMENT
PRÉVENTION DE LA CROISSANCE MICROBIOLOGIQUE DANS UN ÉQUIPEMENT DE TRAITEMENT AYANT UN ÉLÉMENT ROTATIF

(30) Priority: 29.08.2023 EP 23193833
(43) Date of publication of application: 05.03.2025
(73) Proprietor: EPFF Electrical Pipe For Fluid transport AB, 736 32 Kungsör (SE)
(72) Inventor: ANDERSSON, Thomas, 736 32 KUNGSÖR (SE)
(74) Representative: Brann AB

(56) References cited:
- WO-A1-2021/069395
- FR-A1- 2 697 443
- GB-A- 201 907
- US-A1- 2019 249 815

## Description

### Technical field

The technology proposed herein relates to the prevention of microbiological growth in food processing equipment.

### Background

Microbiological growth in food processing equipment is a recognized problem, for example in diary processing. The feed that is processed have a high nutrient content and the rate of microbiological growth in the equipment is typically high. Extensive biofilms can form in a few hours. Frequent cleaning is typically required to prevent a drop in quality of the produced product or in the performance of the equipment. For example, microbiological growth may contaminate the produced product, or it may obstruct the flow of the fluid within the equipment. Typically, production must be interrupted for cleaning and chemicals are commonly used, which contributes to a decreased efficiency and increased production costs.

Microbiological growth is particularly a problem in equipment that that cannot easily be disassembled for manual cleaning. Equipment having rotating components, such as centrifuge pumps, extruders, rotary-type positive-displacement pumps, and mixers are typically difficult to disassemble.
US2019/249815A1 discloses a system for preventing microbiological growth in a piping system.

### Object of the proposed technology

The proposed technology aims at preventing or reducing microbiological growth, such as the formation of biofilms, in food processing equipment. A particular object of the proposed technology is to reduce microbiological growth in equipment having a rotating element.

### Summary

In a first aspect of the proposed technology, a system is proposed that comprises: a housing, or container, and an electric power source, or electric power supply. The housing is electrically conductive and arranged to, or configured to, hold, or contain, a fluid, the power source is arranged to supply an electric current, and/or an electric potential, and the housing and the electric power source are operationally coupled in an electric circuit arranged to lead the current from the electric power source through, or via, the housing, and/or to establish the potential over the housing. The electric current, and/or the electric potential, is configured to inhibit, reduce, or prevent, microbiological growth within the housing, or on the housing.

The system further comprises: a rotating element, or rotary element, wherein the rotating element is located at least partly within the housing. It is understood that the rotating element may be arranged to rotate relative to the housing. It is further understood that the rotating element may rotate around a rotating-element axis, or first rotational axis. It is further understood that the rotating element and the housing may be electrically coupled in an electric network, and that the electric network may form part of the electric circuit. More generally, the rotating element may form part of the electric circuit. It is further understood that the rotating element may be electrically conductive, and that the electric current, and/or the electric potential, may be further configured to inhibit, reduce, or prevent microbiological growth on the rotating element.

It is understood that the housing is a static housing, or stationary housing. It is understood that a static housing does not move in use. The system may further comprise a support structure that fixedly supports the housing. The housing may be electrically connected to, or coupled to, the support structure. Alternatively, the housing may be electrically insulated from the support structure, or from the surroundings. This may contribute to an improved control of the electric current in the housing, and in extension to an improved reduction of microbiological growth.

It is understood that the intention of the system is to prevent microbiological growth in the housing and on the rotating element. It is further understood that the housing and the rotating element may form part of a food processing equipment.

It is further understood that the housing has a portion that is exposed to the fluid, and the electric circuit may be arranged to lead the current from the electric power source through, or via, the portion exposed to the fluid. It is further understood that the current is adapted to inhibit microbiological growth on the housing or on the portion of the housing exposed to the fluid.

It is specified that the electric circuit is arranged to lead the current through the housing. It is understood that the housing extends in all dimensions and that the current may be evenly or unevenly distributed in the housing. It is further understood that at least a portion of the housing forms part of the electric circuit. This means that the current passes through, or via, this portion. The complete housing may form part of the circuit, which means that the current passes through the complete housing.

Throughout these specifications, if a first element is electrically coupled to a second element there may be further conductive elements between them, and if a first element is electrically connected to a second element there are no conductive elements between them.

The fluid may be susceptible to microbiological growth. The fluid may contain water. The fluid may be a liquid, such as raw milk or raw juice. It is understood that the fluid may contain solid particles. The fluid may be a paste, or a minced or pureed product.

The electric circuit may be connected to ground. Worded differently, the housing and/or the rotating element may be electrically coupled to the power source via ground, or the electric circuit may incorporate ground. Ground may be a signal ground or earth ground. For example, ground may incorporate an electrically conductive structure connected to and supporting the housing.

The power source may have a first terminal and a second terminal. The first terminal may supply the current or the potential and the second terminal may be coupled, or connected, to ground, or vice versa. In this context, it is understood that the potential is relative to ground. The power source may be a single-phase power source. This means that it has a single terminal supplying the current or the potential, for example by the first terminal.

The current may be an alternating current. An alternating current is understood to periodically change direction. The current may have a square waveform. The current may be below 10 mA, below 1 mA, or below 0.1 mA. The current may be greater than 0.01 mA. The current may be in the range 10 mA to 1 mA, 1 mA to 0.1 mA, or 0.1 mA to 0.01 mA. Here, the current is understood as the absolute maximum over a period. It has been found that this current is sufficient for inhibiting microbiological growth. The alternating current may have a frequency below 100 Hz, below 10 Hz, or below 1 Hz. The frequency may be greater than 0.1 Hz. The alternating current may be supplied at a peak voltage below 120 V, in the range 10 V to 100 V, or in the range 20 V to 90 V, or in the range 30 V to 70 V.

Alternatively to the current being an alternating current, the current may be a direct current. A direct current is understood to have a constant direction. The direct current may be a pulsed current. It is understood that no current is supplied between pulses. The pulsed current may have a regular pulse frequency. The pulse frequency may be below 100 Hz, below 10 Hz, or below 1 Hz. The pulse frequency may be greater than 0.1 Hz. The direct current may have a duty cycle in the range 60% to 40%, or about 50%. For example, the current may be supplied with a pulse length of 500 ms with a pulse separation of 500 ms. The pulsed current may be supplied at a peak voltage below 120 V, in the range 10 V to 100 V, or in the range 20 V to 90 V, or in the range 30 V to 70 V.

It is specified that the housing is electrically conductive. The housing may be of metal, such as steel. The housing may form, or enclose, an inner space for holding, or retaining, the fluid. The housing may have a wall forming the inner space for holding the fluid. It is understood that the wall is exposed to the fluid. The wall may be electrically conductive. For example, the wall may be of metal. It is understood that the housing, or wall, may be composed of several components that are connected, or joined, together. The wall may be, or form part of the abovementioned portion of the housing.

The housing may have, or form, an inlet, or feed nozzle, for receiving, or introducing, the fluid into the housing. The housing may have, or form, an outlet, or discharge nozzle, for discharging, or dispelling, the fluid from the housing. The inlet and the outlet may be located at opposite sides, or ends, of the housing. It is understood that the housing may be exposed to the fluid between the inlet and the outlet. The electric circuit may be connected to the housing at the inlet and at the outlet. This contributes to inhibit microbiological growth between the inlet and the outlet. If the inlet and the outlet are located at opposite sides of the housing, microbiological growth in the complete housing may be inhibited.

The system may further comprise a feed pipe connected to the inlet of the housing and arranged to, or configured to, lead the fluid to the inlet. Similarly, the system may further comprise a discharge pipe connected to the outlet of the housing and arranged to, or configured to, lead the fluid from the outlet.

The feed pipe and/or the discharge pipe may be electrically conductive. For example, they may be of metal, such as steel. The feed pipe and/or the discharge pipe may be electrically connected to the housing, for example by directly contacting the housing. The feed pipe and/or the discharge pipe may form part of the abovementioned electric circuit. The electric circuit may be arranged to lead the current through, or via, the feed pipe and/or the discharge pipe, and/or to establish the potential over the housing via the feed pipe and/or the discharge pipe. The electric circuit may be arranged to lead the current through and/or to establish the potential over at least a portion of the feed pipe and/or over at least a portion of the discharge pipe. This means that there may be parts of the feed pipe and/or discharge pipe through which the current does not run. The electric circuit may be arranged to lead the current through and/or to establish the potential over the complete feed pipe and/or the discharge pipe.

It is specified that the system comprises a rotating element that is located at least partly within the housing. More specifically, the rotating element may be located at least partly within the inner space. It is understood that the rotating element may comprise several parts. Worded differently, the rotating element may be a composite structure. The parts may be fixed relative to one another. Worded differently, the rotating element may be a rigid structure. Alternatively, the relative positions and/or orientations of the parts may change at a rotation of the rotating element around the rotating-element axis. Worded differently, the rotating element may be a dynamic or deformable structure. It is further understood that that the rotating-element axis may be fixed relative to the housing. Alternatively, the rotating-element axis may be arranged to change position and/or orientation relative to the housing at a rotation of the rotating element around the rotating-element axis, for example by a precession. Worded differently, the rotating-element axis may be arranged to precess.

It is understood that the rotating element may be exposed to the fluid within the housing. The rotating element may extend from within the housing to outside the housing. The rotating element may in part be located within the abovementioned inner space formed by the housing. It may further be accessible from outside the housing. It is understood that the rotating element may be a rigid self-supporting structure. It is understood that the rotating element may conform to the housing, or more specifically to the wall formed by the housing.

It is specified that the housing may have an inlet for receiving the fluid into the housing. Alternatively, the rotating element may form an inlet for introducing the fluid into the housing, or in the inner space formed by the housing. The inlet may be located within the housing or in the inner space.

The rotating element may be, or comprise, a rotating-element shaft. It is understood that the rotating-element shaft may extend from within the housing to outside the housing. It is further understood that it may be arranged to rotate around the rotating-element axis relative to the housing. The rotating-element shaft may be centred on the rotating-element axis. The rotating-element shaft may be a driven shaft, or transmission shaft. For example, the system may comprise a rotating-element motor, wherein the rotating-element motor is coupled to and arranged to rotate the rotating-element shaft. It is understood that the rotating-element motor may be connected directly to the rotating-element shaft, or that it may be connected to the rotating-element shaft via additional components, for example via additional shafts and joints. For example, the rotating-element shaft may be fixed to the rest of the rotating element.

It is specified that the rotating element may be electrically conductive. For example, the rotating element may be of metal, such as steel. It is further specified that the rotating element may form part of the electric circuit. This way, the electric current, and/or the electric potential, is configured to inhibit, reduce, or prevent microbiological growth on the rotating element. The electric circuit may be arranged to lead the current through, or via, both the housing and the rotating element, and/or to establish the potential over the housing and the rotating element. The electric circuit may be arranged to lead the current through and/or to establish the potential over at least a portion of the housing and/or over at least a portion of the rotating element. This means that there may be parts of the housing and/or the rotating element through which the current does not run. The electric circuit may be arranged to lead the current through and/or to establish the potential over the complete housing and/or the complete rotating element. Worded differently, the housing and/or rotating element may have exposed portions, wherein the housing is exposed to the fluid at the exposed portions. The electric circuit may be arranged to lead the current through and/or to establish the potential over the exposed portions.

It is specified that the rotating element and the housing are electrically coupled in an electric network, and that the electric network forms part of the electric circuit. The electric network may be arranged to distribute the current between the housing and the rotating element. For example, the housing and the rotating element may be arranged in parallel in the electric network. This means that in the electric network, a first portion of the current is conducted through, or via, the housing and a second portion of the current is conducted through, or via, the rotating element.

The rotating element, or the rotating-element shaft, may be electrically coupled to the housing. The rotating element, or the rotating-element shaft, may contact the housing in operation. The system may comprise a first internal connector, or first internal connector arrangement, that electrically couples the rotating element, or the rotating-element shaft, to the housing. The system may further comprise a second internal connector, or second internal connector arrangement, that electrically couples the rotating element, or the rotating-element shaft, to the housing. Any of the first internal connector and the second internal connector may be a rotary electrical connector, or electrical rotary joint. Here, rotary electrical connectors are understood to allow a transmission of electrical power or electrical signals from a stationary structure to a rotating structure.

The first internal connector and the second internal connector may be spaced apart along the rotating-element axis. They may be centred on the rotating-element axis.

For example, any of the internal connectors may comprise a slip ring connecting the rotating element, or the rotating-element shaft, and the housing. Additionally or alternatively, any of the internal connectors may comprise a bearing, such as a metal rolling-element bearing or a metal plain bearing connecting the rotating element, or the rotating-element shaft, and the housing. Additionally or alternatively, any of the internal connectors may comprise a metal brush or carbon brush connecting the rotating element, or the rotating-element shaft, and the housing. Additionally or alternatively, any of the internal connectors may comprise a wear ring attached to the rotating element or the housing connecting the rotating element and the housing. Additionally or alternatively, any of the internal connectors may comprise an electrically conductive seal, for example of metal, connecting the rotating element, or the rotating-element shaft, and the housing. It is understood that the seal may be arranged to, or configured to, a inhibit, or prevent, a fluid flow between the rotating element and the housing. A fluid is here understood to included liquids and gases. It is further understood that such a seal is a mechanical seal, or a device that provides a seal at the point of entry or exit of a rotating element.

The rotating element, or the rotating-element shaft, may be connected to the electric circuit via the housing. For example, the abovementioned second portion of the current may pass through, or via, the housing before and/or after passing through, or via, the rotating element. The housing may be connected to the electric circuit via the rotating element, or via the rotating-element shaft. For example, the abovementioned first portion of the current may pass through, or via, the rotating element before and/or after passing through, or via, the housing.

The system may further comprise: a first connector, or first connector arrangement, wherein the housing is electrically coupled to the power source via the first connector. The first connector may be connected to the housing or the rotating element. This means that the first connector forms part of the abovementioned electric circuit. If the first connector is connected to the rotating element, the first connector may be a rotary electrical connector. If the first connector is connected to the housing, the first connector may be a static connector. The system may further comprise: a second connector, or second connector arrangement, wherein the housing is electrically coupled to the power source via the second connector. The second connector may be connected to the housing or the rotating element. This means that the second connector forms part of the abovementioned electric circuit. If the second connector is connected to the rotating element, the second connector may be a rotary electrical connector. If the second connector is connected to the housing, the second connector may be a static connector. The abovementioned support structure may constitute, or form, the first connector or second connector, and the support structure may be connected to ground.

The first connector and the second connector may be positioned on opposite sides of the housing, or the inner space. The abovementioned inlet and/or outlet may be located between the first connector and the second connector. This contributes to inhibit microbiological growth withing a greater part of the housing.

The abovementioned first internal connector and/or second internal connector may be located between the first connector and the second connector, for example along a direction aligned with the rotating-element axis. This contributes to a inhibit microbiological growth over a greater part of the rotating element.

The system may further comprise an electric first wire, and the first connector may be connected to the power source by the first wire. The first wire may be connected to the abovementioned first terminal of the power source. Alternatively, the first connector and the power source may be connected via ground, for example via the abovementioned support structure. The system may further comprise an electric second wire, and the second connector may be connected to the power source by the second wire. The second wire may be connected to the abovementioned second terminal of the power source. Alternatively, the second connector and the power source may be connected via ground, for example via the abovementioned support structure. It is understood that the first connector and the second connector may not both be connected to the power source via ground.

It is specified above that the housing is a static housing. The first connector may be connected to the housing. Similarly, the second connector may be connected to the housing. In this configuration, any of the first connector and the second connector may be a static connector, or a fixed connector. This means that the connector cannot move relative to the housing. For example, the static connector may be a ring lug connector secured to the housing by a screw.

It is further specified above that the system may comprise a rotating element that is arranged to rotate around a rotating-element axis. If both the first connector and the second connector are connected to the housing, the current may then be distributed to the rotating element via the abovementioned first internal connector and second internal connector. The first connector and the second connector may be spaced apart along the rotating-element axis. This contributes to a inhibit microbiological growth over a greater part of the rotating element, particularly if the abovementioned first internal connector and second internal connector are present and spaced apart along the rotating-element axis.

It is specified above that the system may comprise a feed pipe and/or a discharge pipe. The first connector may be connected to feed pipe. Similarly, the second connector may be connected to the discharge pipe. This contributes to reduce microbiological growth also in the feed pipe and/or discharge pipe.

In these configurations, any of the first connector and the second connector may be a static connector, or a fixed connector. For example, the static connector may be a ring lug connector secured by a screw to the housing, feed pipe, or discharge pipe, or the static connector may be a clamp attached to the feed pipe or discharge pipe.

It is specified above that the housing is a static housing and that the system additionally comprises a rotating element. The first connector may be connected to the rotating element, or to the rotating-element shaft. Similarly, the second connector may be connected to the rotating element, or to the rotating-element shaft. Any of the first connector and the second connector may connect to the rotating element outside the housing, for example to the rotating-element shaft. With this configuration, any of the first connector and the second connector may be a rotary electrical connector, or an electrical rotary joint. If both the first connector and the second connector are connected to the rotating element, the current may then be distributed to the housing via the abovementioned first internal connector and second internal connector. The first connector and the second connector may be spaced apart along the rotating-element axis, or more precisely along a direction aligned with the rotating-element axis. This contributes to a inhibit microbiological growth over a greater part of the rotating element.

For example, the rotary electrical connector may comprise a slip ring connecting to the rotating element, or to the rotating-element shaft. Additionally or alternatively, it may comprise a bearing, such as a metal rolling-element bearing or a metal plain bearing connecting to the rotating element, or to the rotating-element shaft. For example, the bearing may have an inner race connected to the rotating element, or to the rotating-element shaft, and an outer race connected to the abovementioned support structure. Additionally or alternatively, the rotary electrical connector may comprise a metal brush or carbon brush connecting to the rotating element, or to the rotating-element shaft. Additionally or alternatively, it may comprise a wear ring connecting to the rotating element, or to the rotating-element shaft. Additionally or alternatively, it may comprise an electrically conductive seal, for example of metal, connecting to the rotating element, or to the rotating-element shaft.

It is specified above that the housing is a static housing and that the system comprises a rotating element. The system may further comprise: an additional rotating element, or additional rotary element, wherein the additional rotating element is located at least partly within the housing, or in the inner space. Worded differently, the rotating element and the additional rotating element may be at least partly located in the same inner space. It is understood that the additional rotating element may be exposed to the fluid within the housing. It is understood that the additional rotating element may be arranged to rotate relative to the housing. The additional rotating element may be arranged to rotate around an additional rotating-element axis, or additional first rotational axis. It is understood that the rotating-element axis and the additional rotating-element axis may be parallel and spaced apart. Worded differently, the additional rotating-element axis may be aligned with and located beside the rotating-element axis.

The additional rotating element may then be, or comprise, an additional rotating-element shaft. It is understood that the additional rotating-element shaft may extend from within the housing to outside the housing. It is further understood that it may be arranged to rotate around the additional rotating-element axis relative to the housing. The additional rotating-element shaft may be centred on the additional rotating-element axis. It is understood that the additional rotating-element shaft may be parallel with the rotating-element shaft.

It is specified that the additional rotating element may be arranged to rotate around an additional rotating-element axis. Alternatively, the additional rotating element may be arranged to rotate around the rotating-element axis relative to the housing. This means that the rotation of the rotating element and the rotation of the additional rotating element are coaxial. The additional rotating-element shaft may be arranged to rotate around the rotating-element axis relative to the housing. The additional rotating-element shaft may be centred on the rotating-element axis. It is understood that the additional rotating-element shaft may be parallel with the rotating-element shaft. It is further understood that that the additional rotating-element shaft may be coaxial with the rotating-element shaft.

The additional rotating element may be a rigid structure. For example, the additional rotating-element shaft may be fixed to the rest of the additional rotating element.

The additional rotating-element shaft may be a driven shaft, or transmission shaft. For example, the system may comprise an additional rotating-element motor, wherein the additional rotating-element motor is coupled to and arranged to rotate the additional rotating-element shaft.

Alternatively to the additional rotating-element shaft being a driven shaft, the system may comprise a gear train, or gear set, operationally connecting the additional rotating element to the rotating element, or more specifically the additional rotating-element shaft to the rotating-element shaft. Worded differently, the gear train is arranged to rotate the additional rotating element at a rotation of the rotating element, or more specifically to rotate the additional rotating-element shaft at a rotation of the rotating-element shaft. The gear train may be arranged to rotate the additional rotating-element shaft synchronously with, or at the same rate as, the rotating-element shaft. Worded differently, the gear train may be a timing gear with gear ratio of one. The gear train may be arranged to rotate the rotating-element shaft and the additional rotating-element shaft in opposite directions. More specifically, the gear train may be a two-gear gear train. The gear train may comprise a gear connected, or fixed, to the rotating-element shaft and an additional gear connected, or fixed, to the additional rotating-element shaft, wherein the gear and the additional gear mesh with one another. It is understood that the gear is centred on the rotating-element axis and that the additional gear is centred on the additional rotating-element axis. For example, the gear and the additional gear may be spur gears or helical gears.

The additional rotating element may be electrically conductive. For example, the additional rotating element may be of metal, such as steel. The additional rotating element, or the additional rotating-element shaft, may form part of the abovementioned electric circuit. This way, the electric current, and/or the electric potential, is further configured to inhibit, reduce, or prevent microbiological growth on the additional rotating element. The electric circuit may be arranged to lead the current through, or via, the additional rotating element, and/or to establish the potential over the additional rotating element. The electric circuit may be arranged to lead the current through and/or to establish the potential over at least a portion of the additional rotating element. This means that there may be parts of the additional rotating element through which the current does not run. The electric circuit may be arranged to lead the current through and/or to establish the potential over the complete additional rotating element.

It is specified that the rotating element and the housing may be electrically coupled in an electric network. The additional rotating element may further be electrically coupled in the electric network.

The rotating element and the additional rotating element may be arranged in parallel in the electric circuit. The electric network may be arranged to distribute the current between the housing, the rotating element, and the additional rotating element. For example, the housing, the rotating element, and the additional rotating element may be arranged in parallel in the electric network. This means that in the electric network, a first portion of the current is conducted through, or via, the housing, a second portion of the current is conducted through, or via, the rotating element, and a third portion of the current is conducted through, or via, the additional rotating element.

The rotating element and the additional rotating element may be arranged in series in the electric circuit. The electric network may be arranged to distribute the current between the housing, and the combined rotating element and additional rotating element. For example, the housing, and the combined rotating element and the additional rotating element may be arranged in parallel in the electric network. This means that in the electric network, a first portion of the current is conducted through, or via, the housing, and a second portion of the current is conducted through, or via the combined rotating element and additional rotating element.

The gear train may electrically couple, or electrically connect the rotating-element shaft and the additional rotating element shaft, or more generally the rotating element and the additional rotating element. Worded differently, the gear train may be electrically coupled in the electric network, or it may form part of the electric circuit.

The additional rotating element, or the additional rotating-element shaft, may be electrically coupled to the rotating element, or more specifically to the rotating-element shaft, and/or to the housing. The additional rotating element, or more specifically the additional rotating-element shaft, may contact the rotating-element, or more specifically the rotating element shaft, and/or the housing in operation. The system may comprise a third internal connector, or third internal connector arrangement, that electrically couples the additional rotating element, or the additional rotating-element shaft, to the rotating element, or more specifically to the rotating-element shaft, and/or to housing. The system may further comprise a fourth internal connector, or fourth internal connector arrangement, that electrically couples the additional rotating element, or more specifically the additional rotating-element shaft, to the rotating element, or more specifically to the rotating-element shaft, and/or to the housing. Any of the third internal connector and the fourth internal connector may be a rotary electrical connector, or electrical rotary joint.

It is specified that the additional rotating element may be arranged to rotate around an additional rotating-element axis. The third internal connector and the fourth internal connector may be spaced apart along the additional rotating-element axis. They may be centred on the additional rotating-element axis. Alternatively, with the additional rotating element arranged to rotate around the rotating-element axis, the third internal connector and the fourth internal connector may be spaced apart along the rotating element-axis, and they may be centred on the rotating-element axis.

Different types of internal connectors are described above. The third internal connector and the fourth internal connector may be any of the described types. For example, the third internal connector may be a slip ring connecting the additional rotating element and the housing and the fourth internal connector may be a bearing, such as a metal rolling-element bearing or a metal plain bearing connecting the additional rotating-element shaft and the housing.

The additional rotating element, or the additional rotating-element shaft, may be connected to the electric circuit via the housing. For example, the abovementioned third portion of the current may pass through, or via, the housing before and/or after passing through, or via, the additional rotating element. The housing may be connected to the electric circuit via the additional rotating element, or via the additional rotating-element shaft.

The housing and the rotating element may form part of a centrifugal pump. Wording the first aspect of the proposed technology differently, the system comprises: an electric power source, or electric power supply, and a centrifugal pump having a housing and a rotating element. The housing is electrically conductive and arranged to, or configured to, hold, or contain, a fluid. The power source is arranged to supply an electric current, and/or an electric potential, and the housing and the electric power source are operationally coupled in an electric circuit arranged to lead the current from the electric power source through, or via, the housing, and/or to establish the potential over the housing. The electric current, and/or the electric potential, is configured to inhibit, reduce, or prevent microbiological growth within the housing, or on the housing. As specified above, it is understood that the fluid may be a liquid.

It is understood that the system may have any of the features described above. For example, the housing may be a static housing and the rotating element may have a rotating-element shaft. The outlet may be located off-axis relative to the rotating-element axis. The inlet may be centred on the rotating-element axis. The housing, or the inner space, may form a volute or curved funnel with cross-section that increases towards the outlet. Worded differently, the housing may form, or constitute, a volute casing. The rotating element may comprise an impeller, wherein the impeller is attached to, or fixed to, the rotating-element shaft and arranged to, or configured to, generate a flow of the fluid from the inlet to the outlet at a rotation around the rotating-element axis. It is understood that the impeller is located in the volute or volute casing.

The first internal connector may connect the impeller to the housing. For example, the first internal connector may be a wear ring attached to the impeller or the housing that connects the impeller and the housing. The second internal connector may be a seal and/or bearing connecting the rotating-element shaft and the housing. The impeller may be located between the first internal connector and the second internal connector. This contributes to inhibit microbiological growth on the complete impeller and any parts of the rotating-element shaft extending into the inner space of the housing.

The first connector may connect to the housing and the second connector may connect to the rotating-element shaft, for example outside the housing. The first internal connector may be located axially between the first connector and the second connector relative to the rotating-element axis. The impeller may be located axially between the first internal connector and the second connector relative to the rotating-element axis. Preferably, the second connector is coupled to ground.

Alternatively, the first connector may connect to the inlet and the second connector may connect to the outlet, as described above. Alternatively, the system further may comprise a feed pipe connected to the inlet of the housing and a discharge pipe connected to the outlet of the housing, as described above. The first connector may then be connected to the feed pipe and the second connector may then be connected to the discharge pipe.

The impeller may be semi-open, or more specifically have a rear hub plate that supports a plurality of vanes that face the inlet. The impeller may be closed, or more specifically have a rear hub plate and a forward hub plate that supports a plurality of vanes between them and with the forward plate forming an impeller eye facing the inlet. It is understood that the vanes are connected to both the rear hub plate and the forward hub plate, and that the impeller can conduct a current between the rear hub plate to the forward hub plate. The abovementioned first internal connector may connect the forward hub plate to the housing.

The housing and the rotating element may form part of an axial-flow pump. Wording the first aspect of the proposed technology differently, the system comprises: an electric power source, or electric power supply, and an axial-flow pump having a housing and a rotating element. The housing is electrically conductive and arranged to, or configured to, hold, or contain, a fluid. The power source is arranged to supply an electric current, and/or an electric potential, and the housing and the electric power source are operationally coupled in an electric circuit arranged to lead the current from the electric power source through, or via, the housing, and/or to establish the potential over the housing. The electric current, and/or the electric potential, is configured to inhibit, reduce, or prevent microbiological growth within the housing, or on the housing. As specified above, it is understood that the fluid may be a liquid.

It is understood that the system may have any of the features described above. For example, the housing is a static housing and the rotating element may have a rotating-element shaft. The inlet and outlet may be centred on the rotating-element axis. Worded differently, the housing may form, or the inner space may outline, a straight tube between the inlet and the outlet. Alternatively, the inlet may be centred on the rotating-element axis and the outlet may be located off-axis relative to the rotating-element axis, or the outlet may be centred on the rotating-element axis and the inlet may be located off-axis relative to the rotating-element axis. Worded differently, the housing may form, or the inner space may outline, a bent tube between the inlet and the outlet.

The rotating element may comprise an impeller, wherein the impeller is attached to, or fixed to, the rotating-element shaft and arranged to, or configured to, generate a flow of the fluid from the inlet to the outlet at a rotation around the rotating-element axis. It is understood that the impeller may be propeller-like.

The housing may comprise, or form, an inducer that is located upstream relative to the flow of the fluid. It is understood that the inducer is electrically coupled to the rest of the housing. It is further understood that the inducer may be arranged to, or configured to, increase the pressure of the fluid prior to the fluid reaching the impeller, for example to avoid cavitation. The first internal connector may connect the impeller, or the rotating-element shaft, to the housing, or more specifically to the inducer. Worded differently, the rotating element, or more specifically the impeller, may be coupled in the electric circuit via the inducer, or the inducer may form part of the electric circuit.

The housing may comprise, or form, a diffuser that is located downstream relative to the flow of the fluid, for example if the housing forms a straight tube between the inlet and the outlet. It is understood that the diffuser is electrically coupled to the rest of the housing. It is further understood that a diffuser may be arranged to, or configured to, reduce the velocity of the fluid after passing the impeller. The second internal connector may connect the impeller, or the rotating-element shaft, to the housing, or more specifically to the diffuser. Worded differently, the rotating element, or more specifically the impeller, may be coupled in the electric circuit via the diffuser, or the diffuser may form part of the electric circuit. The impeller may be located axially between the first internal connector and the second internal connector relative to the rotating-element axis.

The first connector may connect to the housing and the second connector may connect to the rotating-element shaft, for example outside the housing. The first internal connector may be located axially between the first connector and the second connector relative to the rotating-element axis. The impeller may be located axially between the first internal connector and the second connector relative to the rotating-element axis. Preferably, the second connector is coupled to ground.

The housing and the rotating element may form part of an extruder. Wording the first aspect of the proposed technology differently, the system comprises: an electric power source, or electric power supply, and an extruder having a housing and a rotating element. The housing is a static housing, electrically conductive, and arranged to, or configured to, hold, or contain, a fluid. The power source is arranged to supply an electric current, and/or an electric potential, and the housing and the electric power source are operationally coupled in an electric circuit arranged to lead the current from the electric power source through, or via, the housing, and/or to establish the potential over the housing. The electric current, and/or the electric potential, is configured to inhibit, reduce, or prevent microbiological growth within the housing, or on the housing. The fluid may be a paste, minced product, or pureed product.

It is understood that the system may have any of the features described above. For example, the housing is a static housing and the rotating element may have a rotating-element shaft. Worded differently, the housing may be a casing or a barrel. The inlet may be, or have, a feed nozzle or a hopper. It may be located off-axis relative to the rotating-element axis. The outlet may be a discharge nozzle or die. It may be centred on the rotating-element axis. The housing may be a casing or a barrel. The rotating element may further comprise a rotor, or screw, wherein the rotor is attached to, or fixed to, the rotating-element shaft and arranged to, or configured to, generate a flow of the fluid, or to push the fluid, from the inlet to the outlet at a rotation around the rotating-element axis. The rotor may be centred on the rotating-element axis. The rotor may contact and establish an electric connection to the housing, or the wall of the housing. It is understood that this may be in operation of the extruder.

The rotor may contact and establish an electric connection to the housing, or the wall of the housing. The first internal connector may connect the rotating-element shaft to the housing. For example, the first internal connector may be a bearing and a seal that connects the rotating-element shaft and the housing. The first connector may connect to the housing and the second connector may connect to the housing. The first internal connector and the rotor may be located between the first connector and the second connector. The second connector may be located at the die. Preferably, the second connector is coupled to ground.

Additionally or alternatively to the rotor contacting the housing, the abovementioned second internal connector may connect the rotor to the housing. The rotor may be located between the first internal connector and the second internal connector. Worded differently, the first internal connector may be located at a rear end of the rotor and the second internal connector may be located at an opposite forward end of the rotor. Here, rear and forward are relative to the flow of the fluid generated by the rotor. The housing may form a nacelle, or support structure, within the inner space, the second internal connector may be a bearing centred on the rotating-element axis that connects the rotor to the nacelle, and in extension to the rest of the housing.

The housing and the rotating element and the additional rotating element may form part of a rotary-type positive-displacement pump. Wording the first aspect of the proposed technology differently, the system comprises: an electric power source, or electric power supply, and a rotary-type positive-displacement pump having a housing and a rotating element. The power source is arranged to supply an electric current, and/or an electric potential, and the housing and the electric power source are operationally coupled in an electric circuit arranged to, or configured to, lead the current from the electric power source through, or via, the housing, and/or to establish the potential over the housing. The electric current, and/or the electric potential, is configured to inhibit, reduce, or prevent microbiological growth within the housing, or on the housing. As specified above, it is understood that the fluid may be a liquid.

It is specified that the system may further comprise an additional rotating element. The additional rotating element may form part of the rotary-type positive-displacement pump. Worded differently, the positive-displacement pump may further have an additional rotating element.

It is understood that the system may have any of the features described above. For example, the housing is a static housing, the rotating element may have a rotating-element shaft, and, if present, the additional rotating element may have an additional rotating-element shaft. The rotating element and the housing may be electrically coupled in an electric network that forms part of the electric circuit, as described above. If present, the additional rotating element may be coupled in the electric network, or form part of the electric circuit. The housing may have an inlet for receiving fluid into the housing and an outlet for discharging the fluid from the housing.

For example, the rotary-type positive-displacement pump may be a single-screw pump. The rotating element may then comprise, or form, a screw. The screw is attached to, or fixed to, the rotating-element shaft. It is further understood that the screw is arranged to, or configured to, generate a flow of the fluid from the inlet to the outlet. The housing may comprise, or form, a stator, and the screw and the stator are arranged to, or configured to, cooperate and generate a flow of the fluid from the inlet to the outlet. It is understood that the flow is generated at a rotation of the rotating element relative to the housing.

Alternatively, the rotary-type positive displacement pump may be a vane pump. The rotating element may then comprise a rotor that has, or comprises, vanes. The rotor is attached to, or fixed to, the rotating-element shaft. The vanes may have a variable length and/or be biased, or tensioned, to maintain contact with the housing. It is understood that the vanes may extend radially relative to the rotating-element shaft or axis. It is further understood that the vanes are arranged to, or configured to, generate a flow of the fluid from the inlet to the outlet at a rotation of the rotor relative to the housing.

Alternatively, the rotary-type positive displacement pump may be a lobe pump or a circumferential piston pump, which are further described below.

Alternatively, the rotary-type positive displacement pump may be a gear pump, for example an internal gear pump or an external gear pump. The rotating element may then comprise a gear wheel and the additional rotating element may then comprise an additional gear wheel. It is understood that the gear wheel may be centred on, or coaxial with, the rotating-element shaft or axis, and that the additional gear wheel may be centred on, or coaxial with, the additional rotating-element shaft or axis. It is further understood that the gear wheel is attached to, or fixed to, the rotating-element shaft. It is further understood that the additional gear wheel is attached to, or fixed to, the additional rotating-element shaft. It is further understood that the gear wheel and the additional gear wheel are arranged to, or configured to, cooperate to generate a flow of the fluid from the inlet to the outlet at a rotation of the rotating element and a rotation of the additional rotating element relative to the housing. In the internal gear pump, the gear wheel is an internal gear wheel with gear teeth facing inwards and the additional gear wheel is an external gear wheel with gear teeth facing outwards, or vice versa. In the external gear pump, the gear wheel and the additional gear wheel are both external gear wheels with gear teeth facing outwards. It is further understood that the gear wheel and the additional gear wheel mesh with each other. Worded differently, the gear wheel and the additional gear wheel may be operationally connected. The additional gear wheel may be electrically connected to the gear wheel. The additional gear wheel may be arranged to be driven by the gear wheel.

Alternatively, the rotary-type positive displacement pump may be a multi-screw pump, for example a twin-screw pump or a three-screw pump. The rotating element may then comprise a screw and the additional rotating element may then comprise an additional screw. The screw is attached to, or fixed to, the rotating-element shaft, and the additional screw is attached to, or fixed to, the additional rotating-element shaft. It is understood that the screw may be centred on, or coaxial with, the rotating-element shaft or axis, and that the additional screw may be centred on, or coaxial with, the additional rotating-element shaft or axis. It is further understood that the screw and the additional screw are arranged to, or configured to, cooperate to generate a flow of the fluid from the inlet to the outlet at a rotation of the rotating element and a rotation of the additional rotating element relative to the housing. It is further understood that the screw and the additional screw mesh with each other. Worded differently, the screw and the additional screw may be operationally connected. The additional screw may be electrically connected to the screw. The screw may be a female screw, and the additional screw may be a male screw. The additional screw may be arranged to be driven by the screw.

Alternatively, the rotary-type positive displacement pump may be a shuttle block pump. The rotating element may comprise a rotor, a piston, and a shuttle. The rotor is attached to, or fixed to, the rotating-element shaft. The rotor is centred on the rotating-element axis. The shuttle is rotationally connected to, or rotationally supported by, the housing and centred on a shuttle axis that non-coaxial with, or spaced apart from, the rotating-element axis. It is understood that the shuttle axis and the rotating-element axis are parallel. The piston is slidably supported by the rotor and the shuttle reciprocates the piston relative to the rotor at a rotation of the rotating element, or more precisely at a rotation of the rotor. It is understood that the rotor forms a slot, or cylinder, in which the piston is located. It is further understood that the piston rotates with the rotor. The piston cooperates with the rotor to generate a flow of the fluid from the inlet to the outlet at a rotation of the rotating element. It is understood that the rotation of the shuttle is eccentric relative to the rotating-element axis. It is understood that the piston may be in direct contact with the rotor, and that the shuttle may be in direct contact with the piston. More specifically, the piston may be electrically connected to the rotor, and the shuttle may be electrically connected to the piston and the housing.

Alternatively, the rotary-type positive displacement pump may be a progressing cavity pump, or eccentric screw pump. The rotating element may then comprise, or form, a helical rotor and the housing may comprise, or form, a stator having a helical hole in which the helical rotor is located. The pitch of the helical rotor may be twice the pitch of the helical hole. It is understood that the helical rotor and the stator are arranged to, or configured to, cooperate and generate a flow of the fluid from the inlet to the outlet, and that the flow is generated at a rotation of the rotating element around the rotating-element axis and at a precession of the rotating-element axis relative to the housing. It is further understood that the progressive cavity pump may further comprise a connecting rod that is coupled, or connected, to the rotating-element shaft and the abovementioned rotating-element motor via universal joints to allow the precession of the rotating-element axis. Alternatively to the universal joints, the connecting rod may be flexible. The connecting rod may form part of the abovementioned electric circuit. The helical rotor may be coupled to the electric circuit via the connecting rod. The connecting rod may be electrically coupled, or electrically connected, to the helical rotor. The helical rotor may contact the housing, or more specifically the stator. It is understood that this is at any position and orientation in the rotation of the rotating element around the rotating-element axis and at the precession relative to the housing. The helical rotor may be coupled to the electric circuit via the stator. More specifically, the helical rotor may be electrically connected to the stator.

The housing, the rotating element, and the additional rotating element may form part of a dual-rotor pump. For example, the dual-rotor pump may be a lobe pump or a circumferential piston pump. Wording the first aspect of the proposed technology differently, the system comprises: an electric power source, or electric power supply, and a dual-rotor pump having a housing, a rotating element, and an additional rotating element. The housing is electrically conductive and arranged to, or configured to, hold, or contain, a fluid. The power source is arranged to supply an electric current, and/or an electric potential, and the housing and the electric power source are operationally coupled in an electric circuit arranged to lead the current from the electric power source through, or via, the housing, and/or to establish the potential over the housing. The electric current, and/or the electric potential, is configured to inhibit, reduce, or prevent microbiological growth within the housing, or on the housing. As specified above, it is understood that the fluid may be a liquid.

It is understood that the system may have any of the features described above. For example, the housing is a static housing, the rotating element may have a rotating-element shaft, and the additional rotating element may have an additional rotating-element shaft. The rotating element, the additional rotating element, and the housing may be electrically coupled in an electric network that forms part of the electric circuit. The inlet and the outlet may be located off-axis relative to the rotating-element axis. The rotating element may comprise, or form, a rotor, and the additional rotating element may comprise, or form, an additional rotor.

The rotor and the additional rotor are arranged to, or configured to, cooperate and generate a flow of the fluid from the inlet to the outlet at a rotation of the rotating element around the rotating-element axis and a rotation of the additional rotating element around the additional rotating-element axis. Worded differently, the housing, rotor, and additional rotor may be arranged to, or configured to, cooperate to generate a flow of the fluid from the inlet to the outlet. The flow at the inlet and the outlet may be transverse to, or perpendicular to, the rotating-element axis. The inlet and the outlet may be located on opposite sides of the combined rotating element and additional rotating element, or on opposite sides of the combined rotor and additional rotor.

As mentioned above, the dual-rotor pump may be a lobe pump. This means that the rotor is a lobe rotor, and that the additional rotor is an additional lobe rotor. Worded differently, the rotor may have, or form, a hub and one or more, or a plurality of, lobes that extend from the hub, and the additional rotor may have, or form, an additional hub and one or more, or a plurality of, additional lobes that extend from the additional hub. For example, the rotor may a bi-lobe rotor. More specifically, the rotor may have two lobes that extends from the hub and the additional rotor may have two additional lobes that extend from the additional hub. Alternatively, the rotor may a tri-lobe rotor. More specifically, the rotor may have three lobes that extends from the hub and the additional rotor may have three additional lobes that extend from the additional hub.

Neighbouring lobes may join, or merge at the hub. Similarly, neighbouring additional lobes may join, or merge at the hub. This means that the radial outer part of hub is covered by the lobes and the radial outer part of the additional hub is covered by the additional lobes. It is understood that the lobes and the additional lobes may be arranged such that at least one lobe is located at, or has a close clearance to, at least one additional lobe at any rotational positions of the rotating element and the additional rotating element. It is further understood that simultaneously at least one lobe is located at, or has close clearance to, the wall of housing and at least one additional lobe is located at, or has close clearance to, the wall of housing.

The lobe rotor and the additional lobe rotor may be straight lobe rotors. Worded differently, each of the lobe and the additional lobe may have a profile, or radial cross-section, that does not twist, or spiral, around to the rotating-element axis respective around the additional rotating-element axis. Alternatively, the lobe rotor and the additional lobe rotor may be spiral lobe rotors. Worded differently, each of the lobe and the additional lobe may have a profile, or radial cross-section, that twists, or spirals, around to the rotating-element axis respective around additional rotating-element axis.

As mentioned above, the dual-rotor pump may be a circumferential piston pump. This means that the rotor is a winged rotor, and that the additional rotor is an additional winged rotor. Worded differently, the rotor may have, or form, a hub and one or more wings, or flared protrusions, that extend from the hub, and the additional rotor may have, or form, an additional hub and one or more additional wings, or additional flared protrusions, that extend from the additional hub. For example, the rotors may be single-wing rotors. More specifically, the rotor may have a single wing that extends from the hub and the additional rotor may have a single additional wing that extends from the additional hub. Alternatively, the rotor may a bi-wing rotor. More specifically, the rotor may have two wings that extends from the hub and the additional rotor may have two additional wings that extend from the additional hub.

The hub may be exposed to the inner space, or configured to be exposed to the fluid. This means that the hub is not completely covered by the one or more wings and the additional hub is not completely covered by the one or more additional lobes. It is understood that the one or more wings and the one or more additional wings may be arranged such that a wing is located at, or has a close clearance to, both the wall of the housing and the additional hub, or an additional wing is located at, or has a close clearance to, both the wall of the housing and the additional hub, at any rotational positions of the rotating element and the additional rotating element.

In the dual-rotor pump, the rotating-element shaft may be attached to, or fixed to, the rotor, or more specifically to the hub of the rotor. Similarly, the additional rotating-element shaft may be attached to, or fixed to, the additional rotor, or more specifically to the additional hub of the additional rotor. The rotating-element shaft may extend through the hub of the rotor. Similarly, the additional rotating-element shaft may extend through the additional hub of the additional rotor. As specified above, the housing may form, or enclose, an inner space for holding, or retaining, the fluid. It is understood that the rotor, or the lobes and the hub, and the additional rotor, or the additional lobes and the additional hub, are located within the housing, or more specifically within the inner space.

The rotor and the additional rotor in the dual-rotor pump may be spaced apart, or have a clearance between them. More specifically, the additional lobes or additional wings may be spaced apart from, or have a clearance to, the lobes or the wings. It is understood that this when operating the dual-rotor pump, or at any rotational positions of the rotating element and the additional rotating element. This means that current cannot pass directly between the rotor and the additional rotor, or more specifically between the lobes and the additional lobes, between the wings and the additional hub, or between the additional wings and the hub. The rotor and the housing may be spaced apart, or have a clearance between them. More specifically, the lobes or the wings may be spaced apart from, or have a clearance to, the housing. Similarly, the additional rotor and the housing may be spaced apart, or have a clearance between them. More specifically, the additional lobes or the additional wings may be spaced apart from, or have a clearance to, the housing. It is understood that this when operating the dual-rotor pump, or at rotation of the rotating element and the additional rotating element. This means that current cannot pass directly between the rotor or the additional rotor and the housing.

It is specified above that the system may comprise a first internal connector that electrically couples the rotating element, or the rotating-element shaft, to the housing. It is further specified that the system may further comprise a second internal connector that electrically couples the rotating element, or the rotating-element shaft, to the housing. The rotor may be located between the first internal connector and the second internal connector relative to, or along, the rotating-element axis. This allows for the rotor to form part of the abovementioned electric network with the rotor being spaced apart from the housing, thus contributing to inhibit microbiological growth on the rotor.

It is specified above that the system may comprise a third internal connector that electrically couples the additional rotating element, or the additional rotating-element shaft, to the housing. It is further specified that the system may further comprise a fourth internal connector that electrically couples the additional rotating element, or the additional rotating-element shaft, to the housing. The additional rotor may be located between the third internal connector and the fourth internal connector relative to, or along, the additional rotating-element axis. This allows for the additional rotor to form part of the abovementioned electric network with the additional rotor being spaced apart from the rotor and the housing, thus contributing to inhibit microbiological growth on the additional rotor.

The first internal connector may connect the rotor, or more specifically the hub of the rotor, to the housing. It is specified that the rotating-element shaft may extend through the hub, and the first internal connector may instead connect the rotating-element shaft to the housing. For example, the first internal connector may be a wear ring attached to the hub, the housing, or the rotating-element shaft. The second internal connector may be a seal and/or bearing connecting the rotating-element shaft and the housing.

Similarly, the third internal connector may connect the additional rotor, or more specifically the additional hub of the additional rotor, to the housing. It is specified that the additional rotating-element shaft may extend through the additional hub, and the third internal connector may instead connect the additional rotating-element shaft to the housing. For example, the third internal connector may be a wear ring attached to the additional hub, the housing, or the additional rotating-element shaft. The fourth internal connector may be a seal and/or bearing connecting the additional rotating-element shaft and the housing.

The first internal connector may be located axially between the first connector and the second connector relative to the rotating-element axis, or in a direction along the rotating element axis. Similarly, the third internal connector may be located axially between the first connector and the second connector relative to the rotating-element axis, or in a direction along the additional rotating element axis. The first connector may connect to the housing and the second connector may connect to the rotating-element shaft, for example outside the housing. The rotor may be located axially between the first internal connector and the second connector relative to the rotating-element axis. Similarly, the third connector may connect to the housing and the fourth connector may connect to the additional rotating-element shaft, for example outside the housing. The additional rotor may be located axially between the third internal connector and the second connector relative to the rotating-element axis. Preferably, the second connector is coupled to ground.

Alternatively, the first connector may connect to the inlet and the second connector may connect to the outlet, as described above. Alternatively, the system further may comprise a feed pipe connected to the inlet of the housing and a discharge pipe connected to the outlet of the housing, as described above. The first connector may then be connected to feed pipe and the second connector may then be connected to the discharge pipe.

The housing and the rotating element and the additional rotating element may form part of a mixer, or agitator. Wording the first aspect of the proposed technology differently, the system comprises: an electric power source, or electric power supply, and a mixer having a housing and a rotating element. The power source is arranged to supply an electric current, and/or an electric potential, and the housing and the electric power source are operationally coupled in an electric circuit arranged to lead the current from the electric power source through, or via, the housing, and/or to establish the potential over the housing. The electric current, and/or the electric potential, is configured to inhibit, reduce, or prevent microbiological growth within the housing, or on the housing. As specified above, it is understood that the fluid may be a liquid.

It is specified that the system may further comprise an additional rotating element. The additional rotating element may form part of the mixer. Worded differently, the mixer may further have an additional rotating element.

It is understood that the system may have any of the features described above. For example, the housing is a static housing, the rotating element may have a rotating-element shaft, and the additional rotating element may have an additional rotating-element shaft. The rotating element and the housing may be electrically coupled in an electric network that forms part of the electric circuit, as described above. The housing may have an inlet for receiving fluid into the housing, an outlet for discharging the fluid from the housing, and/or a combined inlet and outlet for receiving fluid into the housing and discharging fluid from the housing. It is understood that the inlet and/or outlet may be sealable, effectively turning the housing into a container for the fluid.

A mixer is understood to be configured for agitating a fluid, for example to mix two fluid components, or to mix a fluid component with a solid particulate component. The rotating element may comprise an agitator, or rotor. It is understood that the agitator is configured to, or intended to, agitate, or stir, the fluid held, or contained, by the housing at a rotation of the rotating element.

As specified above, the system may comprise a first internal connector that electrically couples the rotating element, or the rotating-element shaft, to the housing, and a second internal connector that electrically couples the rotating element, or the rotating-element shaft, to the housing. The agitator may be connected to, or attached to, the rotating element shaft, for example between the first internal connector and the second internal connector. It is understood that the first internal connector and the second internal connector may be located at opposite sides of the housing, or of the inner space. The agitator may form part of the electric circuit. More specifically, the agitator may be coupled in parallel with the rotating-element shaft.

It is understood that the rotating-element shaft may be composed of a single shaft section. Alternatively, the rotating-element shaft may be composed of a first shaft section and a second shaft section that are spaced apart and interconnected by the agitator. This way the first shaft section, the agitator, and the second shaft section are coupled in series. More generally, the agitator may be coupled in series with the rotating element shaft. It is understood that the first shaft section and the second shaft section may be centred on the rotating-element axis.

It is specified that the system may further comprise an additional rotating element, and that the additional rotating element may be arranged to rotate around the rotating-element axis relative to the housing. It is further specified that the additional rotating element may comprise an additional rotating-element shaft, and that the additional rotating-element shaft may be coaxial with the rotating-element shaft.

The additional rotating element may comprise an additional agitator, or additional rotor. It is understood that the agitator is configured to, or intended to, agitate, or stir, the fluid held, or contained, by the housing at a rotation of the additional rotating element.

As specified above, the system may comprise a third internal connector and a fourth internal connector. The additional agitator may be connected to the rotating element shaft, for example between the third internal connector and the fourth internal connector. The third internal connector may couple the additional rotating element to the rotating element, and the fourth internal connector may couple the additional rotating element to the rotating element. Worded differently, the additional rotating element may be electrically coupled to the housing via the rotating element. Alternatively, the third internal connector may couple the additional rotating element to the housing, and the fourth internal connector may couple the additional rotating element to the housing. Alternatively, the third internal connector may couple the additional rotating element to the housing, and the fourth internal connector may couple the additional rotating element to the rotating element. Alternatively, the third internal connector may couple the additional rotating element to the rotating element, and the fourth internal connector may couple the additional rotating element to the housing.

It is understood that the third internal connector and the fourth internal connector may be located at opposite sides of the housing, or of the inner space. The additional agitator may form part of the electric circuit. More specifically, the additional agitator may be coupled in parallel with the additional rotating-element shaft.

It is understood that the additional rotating element may be arranged to rotate simultaneously to the rotating element. It is further understood that the additional rotating element may be arranged to rotate at a different rate and/or in a different direction than the rotating element. It is understood that the housing may have a top and a bottom. For example, the inlet may be located at the top of the housing and the outlet may be located at the bottom of the housing.

In a second aspect of the proposed technology, which is not a part of the claimed invention, a method of inhibiting, reducing, or preventing microbiological growth in the system, or more specifically in the housing of the system, according to the first aspect is proposed. The method comprises: operating the power source to supply the current.

### Brief description of the drawings

A more complete understanding of the abovementioned and other features and advantages of the technology proposed herein will be apparent from the following detailed description of preferred embodiments in conjunction with the appended drawings, wherein:
Fig. 1 is a schematic view of a system with a centrifugal pump,
Fig. 2 is a schematic-cross section of another centrifugal pump,
Fig. 3a is a schematic view of a system with an extruder,
Fig. 3b is a schematic cross-section of the extruder of Fig. 3a at the rotating-element shaft,
Fig. 4a is a schematic view of a system with another extruder,
Fig. 4b is a schematic cross-section of the extruder of Fig. 4a at the rotating-element shaft,
Figs. 5a and 5b are schematic views of a system with a lobe pump,
Fig. 6 is a schematic view of system with a mixer, and
Fig. 7 is a schematic view of system with another mixer.

### Detailed description

In the figures and the below description, the same reference numerals are used for the same or related features.

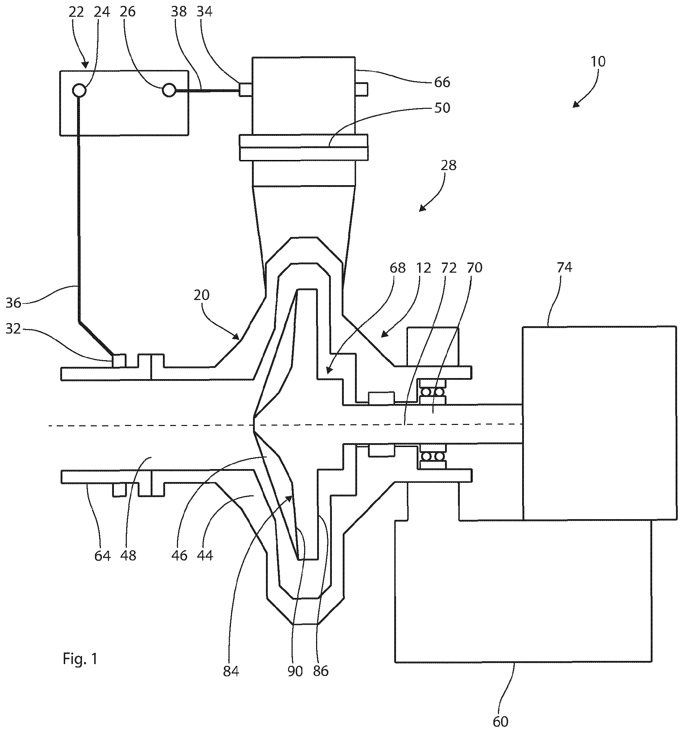

**Fig. 1** shows a system 10 with an electric power source 22 and a centrifugal pump 12 shown in cross section. The centrifugal pump 12 has a housing 20 of steel that is electrically conductive and arranged to hold a liquid.

The housing 20 has an electrically conductive wall 44 that forms an inner space 46 holding the liquid. The wall 44 is exposed to the liquid. The housing 20 has an inlet 48 that can receive the liquid and an outlet 50 that can discharge the liquid from the housing 20. The housing 20 is stationary and the system 10 has a support structure 60 that supports the housing 20. The housing 20 is electrically connected to the support structure 60.

The system 10 has a feed pipe 64 connected to the inlet 48 of the housing 20 by which the liquid can be led to the inlet 48. The system 10 further has a discharge pipe 66 connected to the outlet 50 of the housing 20 by which the liquid can be led from the outlet 50. The feed pipe 64 and the discharge pipe 66 are of steel and electrically connected to the housing 20.

The system 10 further has a rigid rotating element 68 composed of a rotating-element shaft 70 and an impeller 84 that are centred on and can rotate around a rotating-element axis 72. The rotating element 68 is of steel and is electrically conductive. The rotating element 68 is in part located in the inner space 46 and the rotating-element shaft 70 extends from within the housing 20 to outside the housing 20, which means that it is accessible from outside the housing 20. The system 10 further has a rotating-element motor 74 that is connected to and can drive the rotating-element shaft 70.

The inlet 48 is centred on the rotating-element axis 72 and the outlet 50 is located off-axis relative to the rotating-element axis 72. The housing 20 form a volute with cross-section that increases towards the outlet 50. The impeller 84 is located in the volute and is semi-open with a rear hub plate 86 attached to the rotating-element shaft 70 and a plurality of vanes 90 facing the inlet 48. This way, the impeller 84 is arranged to generate a flow of the liquid from the inlet 48 to the outlet 50 at a rotation around the rotating-element axis 72 by the rotating-element motor 74.

The system 10 further has a first connector 32 in the form of a clamp that is attached to the feed pipe 64 and a second connector 34 also in the form of a clamp that is attached to the discharge pipe 66. The system 10 further has an electric first wire 36 connecting the first connector 32 to a first terminal 24 of the power source 22 and an electric second wire 38 connecting the second connector 34 to a second terminal 26 of the power source 22. In an alternative embodiment, the second terminal 26 is connected ground and the discharge pipe 66 is connected to ground.

The first terminal 24 of the power source 22 can supply an alternating current with a square waveform. The current is about 0.5 mA, has a frequency below of 1 Hz, and is supplied at a peak voltage of about 50 V. The supplied current contributes to reduce microbiological growth in the pump 12.

With the power source 22, first wire 36, first connector 32, feed pipe 64, housing 20, discharge pipe 66, second connector 34, and second wire 38 arranged as described above, the housing 20 and the electric power source 22 are operationally coupled in an electric circuit 28 that can establish a first electrical potential over the housing 20 and lead the electric current from the electric power source 22 and through the housing 20. The electric circuit 28 is connected to the housing 20 at the inlet 48 and at the outlet 50. With the described coupling, the housing 20 has a portion that is exposed to the liquid and forms part of the electric circuit. Thus, the electric circuit 28 can lead the current from the power source 22 through the portion exposed to the liquid, thus inhibiting microbiological growth on this portion of the housing 20.

In alternative embodiments, the first connector 32 is connected directly to the housing 20, for example at the inlet 48, and the second connector 34 is connected directly to the housing 20, for example at the outlet 50 or at the rotating-element shaft 70.

The first connector 32 and the second connector 34 are ring lug connectors secured to the housing 20 by screws. In the above embodiments, the housing 20 electrically insulated from the support structure 60. In an alternative embodiment, the system 10 has no second connector. Instead, the second terminal of the power source 22 is connected to ground, the housing 20 is electrically connected to the support structure 60, and the support structure 60 is connected to ground. Effectively, this means that the support structure 60 has the function of the second connector 34 in the electric circuit 28 and it forms part of the electric circuit 28.

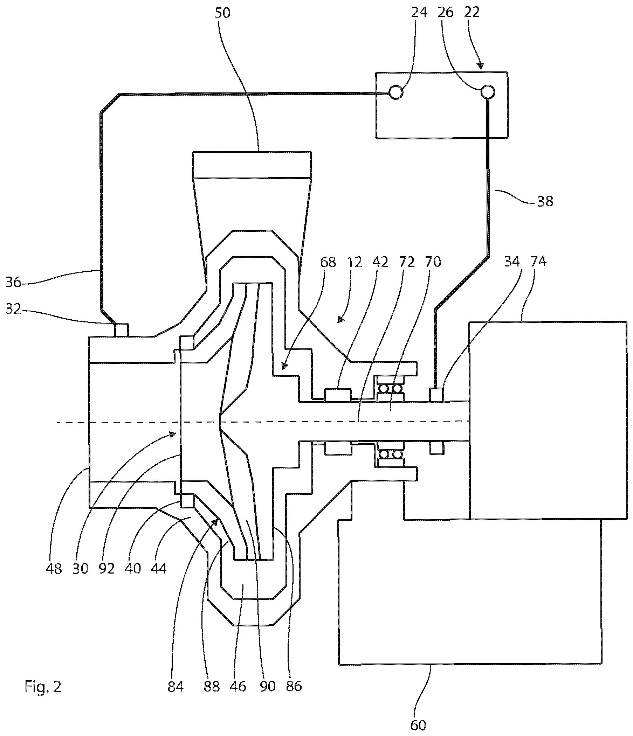

**Fig. 2** shows another embodiment of a system 10 with centrifugal pump 12. The latter is shown in cross section. The system 10 has rotating-element motor 74 and a support structure 60 arranged as described in relation to Fig. 1. The centrifugal pump 12 differs in that the impeller 84 is a closed impeller that further has a forward hub plate 88 that forms an impeller eye 92. The impeller eye 92 faces the inlet 48 and the vanes 90 are supported between and connected to both the rear hub plate 86 and the forward hub plate 88.

The centrifugal pump 12, and in extension the system 10, further has a first internal connector 40 in the form of a wear ring attached to the housing 20. The wear ring 40 is centred on the rotating-element axis 72 and the impeller eye 92. The first internal connector 40 connects and electrically couples the housing 20 and the impeller 84 of the rotating element 68. The centrifugal pump 12, and in extension the system 10, further has a second internal connector 42 in the form of a metal rolling-element bearing that connects and electrically couples the housing 20 and the rotating-element shaft 70 of the rotating element 68.

The first connector 32 is a ring lug connector secured to the housing 20 at the inlet 48 by a screw. The system 10 further differs by the second connector 34 being a slip ring connecting to the rotating-element shaft 70 outside the housing 20. The first internal connector 40 and the second internal connector 42 are located axially between the first connector 32 and the second connector 34 relative to the rotating-element axis 72. The rotating element 68 and the housing 20 are thus electrically coupled in an electric network 30 that forms part of the abovementioned electric circuit 28, and the electric network 30 can distribute the current between the housing 20 and the rotating element 68. In extension, the rotating element 68 forms part of the electric circuit 28, and the electric circuit 28 is arranged to lead the current through both the housing 28 and the rotating element 68.

The first wire 36 connects the first connector 32 to the first terminal 24 of the power source 22 and the second wire 38 connects the second connector 34 to the second terminal 26 of the power source 22, as in Fig. 1. The housing 20 is electrically insulated from to the support structure 60. In an alternative embodiment, the rotating-element shaft 70 and the second terminal 26 are connected to ground via the rotating-element motor 74.

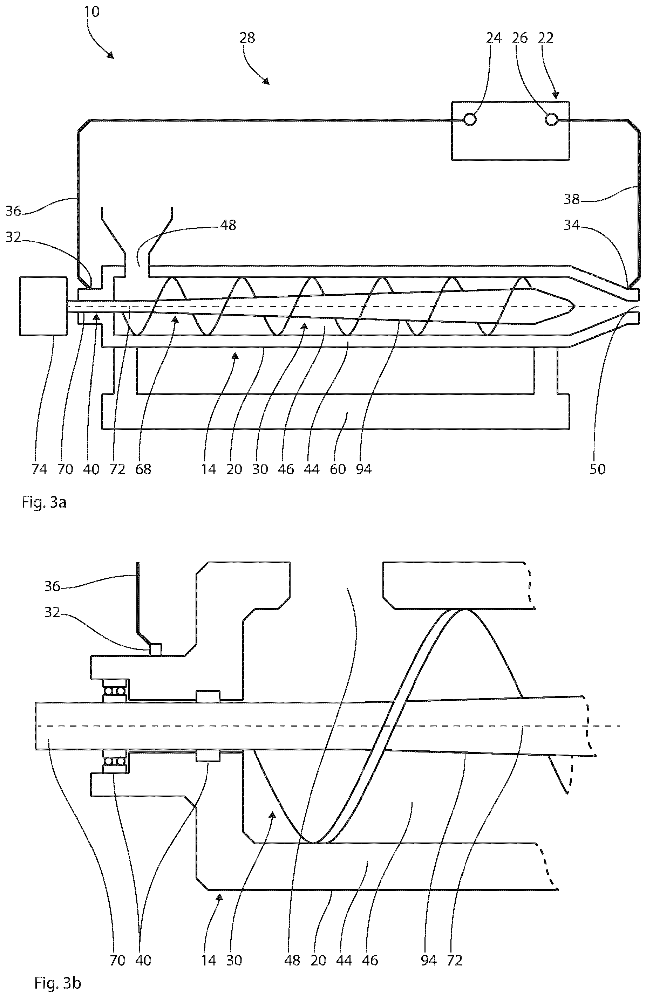

**Fig. 3a** shows an embodiment of a system 10 including an extruder 14 and an electric power source 22. The extruder 14 is shown in cross section and has a housing 20 of steel that is electrically conductive and arranged to hold a fluid. The housing 20 has an electrically conductive wall 44 exposed to the fluid that forms an inner space 46 holding the fluid. The housing 20 has an inlet 48 in the form of a hopper that can receive the fluid and an outlet 50 in the form of a die that can discharge the fluid from the housing 20. The housing 20 is stationary and the system 10 has a support structure 60 that supports the housing 20. The housing 20 is electrically insulated from the support structure 60. In an alternative embodiment it is electrically connected to the support structure 60.

The system 10 further has a rigid rotating element 68 composed of a rotating-element shaft 70 and a rotor 94 that are centred on and can rotate around a rotating-element axis 72. The rotating element 68 is of steel and is electrically conductive. The rotating element 70 is in part located in the inner space 46 and the rotating-element shaft 70 extends from within the housing 20 to outside the housing 20, which means that it is accessible from outside the housing 20.

The inlet 48 is off centre relative to the rotating-element axis 72 and the outlet 50 is centred on the rotating-element axis 72. The system 10 further has a rotating-element motor 74 that is connected to and can drive the rotating-element shaft 70. The rotor 94 pushes the fluid from the inlet 48 to the outlet 50 at a rotation around the rotating-element axis 72.

The system 10 further has a first connector 32 that is attached to the housing at the rotating-element shaft 70 and a second connector 34 attached to the housing at the outlet 50. The first connector 32 and the second connector 34 are ring lug connectors secured to the housing 20 by screws. The system 10 further has an electric first wire 36 connecting the first connector 32 to a first terminal 24 of the power source 22 and an electric second wire 38 connecting the second connector 34 to a second terminal 26 of the power source 22. In an alternative embodiment, the second terminal is connected to ground and the housing is connected to ground via the support structure 60.

The power source 22 can supply an alternating current with a square waveform. The current is about 0.5 mA, has a frequency below of 1 Hz, and is supplied at a peak voltage of 50 V. The supplied current contributes to reduce microbiological growth in the extruder 14.

With the power source 22, first wire 36, first connector 32, housing 20, second connector 34, and second wire 38 arranged as described above, the housing 20 and the electric power source 22 are operationally coupled in an electric circuit 28 that can establish a first electrical potential over the housing 20 and lead an electric current from the electric power source 22 and through the housing 20. The electric circuit 28 is connected to the rotating-element shaft 70 and at the outlet 50.

The extruder 14, and in extension the system 10, further has a first internal connector 40 in the form of a metallic seal and a metal-rolling element bearing that connect the rotating-element shaft 70 and the housing 20, as shown in Fig. 3b. This way, the first internal connector 40 electrically couples the housing 20 and the rotating element 68. The rotor 94 contacts and establish an electric connection with the wall 44 of the housing 20 in operation of the extruder 14. The first internal connector 40 and the rotor 94 are located between the first connector 32 and the second connector 34. The rotating element 68 and the housing 20 are thus electrically coupled in an electric network 30 that forms part of the abovementioned electric circuit 28, and the electric network 30 can distribute the current between the housing 20 and the rotating element 68. In extension, the rotating element 68 forms part of the electric circuit 28, and the electric circuit 28 is arranged to lead the current through both the housing 28 and the rotating element 68.

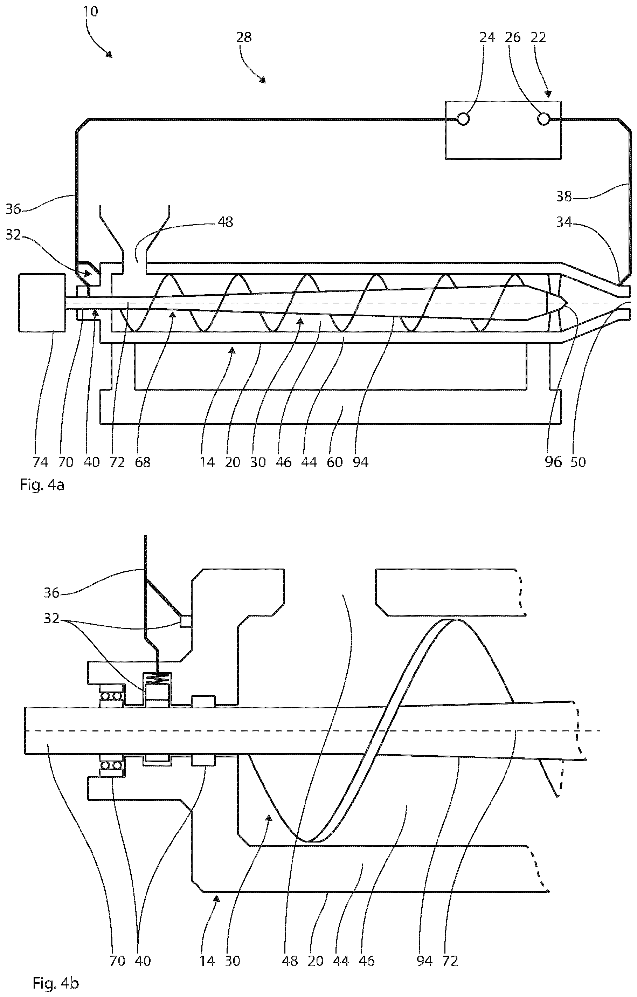

**Fig. 4a** shows another system 10 including an extruder 14 and an electric power source 22. The system 10 differs in that the rotor 94 of the extruder 14 does not contact the wall 44 of the housing 20 in operation. The housing 20 forms a nacelle 96 within the inner space 46, and the second internal connector is a plain bearing centred on the rotating-element axis 72 that connects the rotor 94 to the nacelle 96, and in extension to the rest of the housing 20. The system 10 further differs by the first connector 32 being connected to both the housing 20 by a lug and a screw and to the rotating-element shaft 70 by a carbon brush. The carbon brush is located between the seal and the bearing connecting the housing 20 and the rotating-element shaft 70, as shown in **Fig. 4b****.**

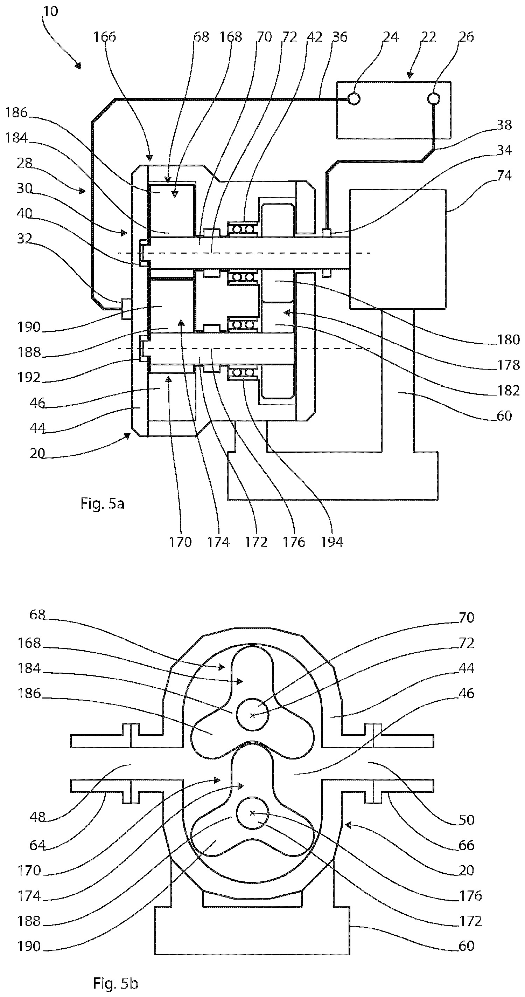

**Fig. 5a** shows a system 10 with an electric power source 22 and a lobe pump 166. In Fig. 5a the lobe pump 166 is shown in cross section along the rotating-element axis 72. **Fig. 5b** shows the lobe pump 166 in cross section transverse to the rotating-element axis 72. The lobe pump 166 has a housing 20 of steel that is electrically conductive and arranged to hold a liquid. The housing 20 has an electrically conductive wall 44 that forms an inner space 46 holding the liquid. The wall 44 is exposed to the liquid. The housing 20 has an inlet 48 that can receive the liquid and an outlet 50 that can discharge the liquid from the housing 20. The housing 20 is stationary and the system 10 has a support structure 60 that supports the housing 20. The housing 20 is electrically connected to the support structure 60.

The system 10 has a feed pipe 64 connected to the inlet 48 of the housing 20 by which the liquid can be led to the inlet 48. The system 10 further has a discharge pipe 66 connected to the outlet 50 of the housing 20 by which the liquid can be led from the outlet 50. The feed pipe 64 and the discharge pipe 66 are of steel and electrically connected to the housing 20.

The system 10 further has a rigid rotating element 68 composed of a rotating-element shaft 70 and a rotor 168 that are centred on and can rotate around a rotating-element axis 72. The rotating element 68 is of steel and is electrically conductive. The rotor 168 is located in the inner space 46 and the rotating-element shaft 70 extends from within the housing 20 to outside the housing 20, which means that the rotating element 68 is in part located in the inner space 46 and that the rotating-element shaft 70 is accessible from outside the housing 20. It also means that the rotor 168 is exposed to the liquid held by the housing 20. The system 10 has a rotating-element motor 74 that is connected to rotating-element shaft 70 and that can rotate the rotating element 68.

The system 10 further has a rigid additional rotating element 170 composed of an additional rotating-element shaft 172 and an additional rotor 174 that are centred on and can rotate around an additional rotating-element axis 176. The additional rotating element 170 is of steel and is electrically conductive. The additional rotor 174 is located in the same inner space 46 as the rotor 168, and the additional rotating-element shaft 172 extends from within the housing 20 to outside the housing 20, which means that the additional rotating element 170 is in part located in the inner space 46 and that the additional rotating-element shaft 172 is accessible from outside the housing 20.

The system 10 further has a two-gear gear train 178 composed of a gear 180 fixed to and centred on the rotating-element shaft 70 and an additional gear 182 fixed to and centred on the additional rotating-element shaft 172. The gear 180 and the additional gear 182 are electrically conductive spur gears of steel that mesh with one another and have the same number of teeth. This way, the gear train 178 operationally connects the additional rotating-element shaft 172 to the rotating-element shaft 70. The gear train 178 electrically couples the rotating-element shaft 70 and the additional rotating element shaft 172, the gear train 178 is arranged to rotate the additional rotating-element shaft 172 at a rotation of the rotating-element shaft 70, and the gear train 178 is arranged to rotate the additional rotating-element shaft 172 synchronously with and in the opposite direction of the rotating-element shaft 70.

The inlet 48 and the outlet 50 are located off-axis relative to the rotating-element axis 72 and the additional rotating-element axis 176, and they are located on opposite sides of the combined rotor 168 and additional rotor 174.

The rotor 168 and the additional rotor 174 are straight tri-lobe rotors. The rotor 168 is a monolithic metal structure, and it has a hub 184 and three lobes 186 that extend from and merges at the hub 184. The additional rotor 174 is a monolithic metal structure, and it has an additional hub 188 and three additional lobes 190 that extend from and merges at the additional hub 188. This means that the lobes 186 jointly cover the radial outer part of the hub 184 and the additional lobes 190 jointly cover the radial outer part of the additional hub 188. As can be seen in Fig. 5b, at least one lobe 186 is located at and has a close clearance to at least one additional lobe 190 at any rotational positions of the rotating element 68 and the additional rotating element 170. Further, at least one lobe 186 is located at and has close clearance to the wall 44 of housing 20 and at least one additional lobe 190 is located at and has close clearance to the wall 44 of housing 20. This way, the rotor 168 and the additional rotor 174 are arranged to cooperate and generate a flow of the fluid from the inlet 48 to the outlet 50 at a rotation of the rotating element 68 around the rotating-element axis 72 and a rotation of the additional rotating element 170 around the additional rotating-element axis 176.

The lobes 186 of the rotor 168 and the additional lobes 190 of the additional rotor 174 are spaced apart and have a clearance between them at any rotational positions of the rotating element 68 and the additional rotating element 170, and no current can pass directly between the rotor 168 and the additional rotor 174.

The rotating-element shaft 70 extends through and is fixed to the hub 184 of the rotor 168, and the additional rotating-element shaft 172 extends through and is fixed to the additional hub 188 of the additional rotor 174.

The system has a first connector 32 in the form of a ring lug connector secured to the housing 20 by a screw and a second connector 34 in the form of a slip ring that is connected the rotating-element shaft 70 outside the housing 20. The system 10 further has an electric first wire 36 connecting the first connector 32 to a first terminal 24 of the power source 22 and an electric second wire 38 connecting the second connector 34 to a second terminal 26 of the power source 22. In an alternative embodiment, the second terminal 26 and the second connector 34 are connected to ground.

The first terminal 24 of the power source 22 can supply an alternating current with a square waveform. The current is about 0.5 mA, has a frequency below of 1 Hz, and is supplied at a peak voltage of about 50 V.

The system 10 further has a first internal connector 40 in the form of a metal wear ring and a second internal connector 42 in the form of a metal rolling-element bearing that are located axially between the first connector 32 and the second connector 34 relative to the rotating-element axis 72. Both the first internal connector 40 and the second internal connector 42 electrically couple the rotating-element shaft 70 to the housing 20. The system 10 further has a third internal connector 192 in the form of a metal wear ring and a fourth internal connector 194 in the form of a metal rolling-element bearing that are located axially between the first connector 32 and the second connector 34 relative to the additional rotating-element axis 176. Both the third internal connector 192 and the fourth internal connector 194 electrically couple the additional rotating-element shaft 172 to the housing 20.

The rotating element 68, the additional rotating element 170, and the housing 20 are thus electrically coupled in an electric network 30. These components together with the power source 22, first wire 36, and second wire 38 form part of an electric circuit 28. This means that the electric network 30 forms part of the electric circuit 28 and can distribute the current between the housing 20, the rotating element 68, and the additional rotating element 170, and the electric circuit 28 is arranged to lead the current through both the housing 28, the rotating element 68, and the additional rotating element 70. This way, the supplied current can contribute to reduce microbiological growth in the lobe pump 166.

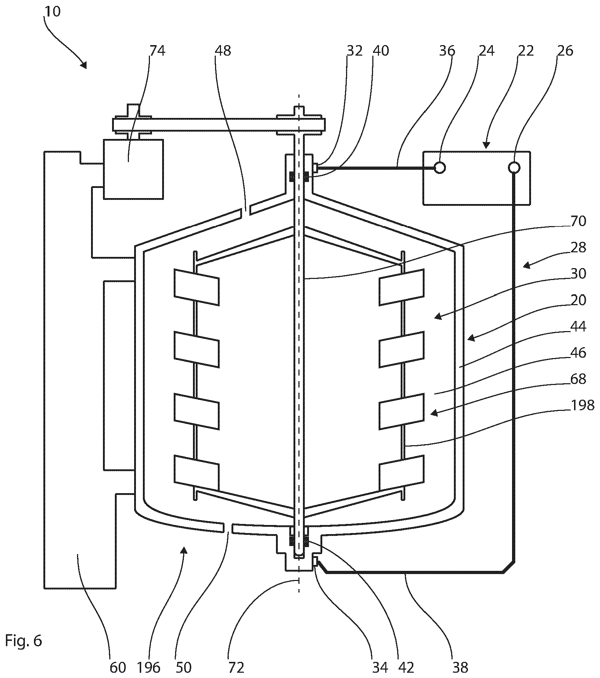

**Fig. 6** shows a system 10 with an electric power source 22 and a mixer 196. The mixer 196 is shown in cross section and has a housing 20 of steel that is electrically conductive and arranged to hold a liquid. The housing 20 has an electrically conductive wall 44 that forms an inner space 46 holding the liquid. The wall 44 is exposed to the liquid. The housing 20 has a sealable inlet 48 that can receive the liquid and a sealable outlet 50 that can discharge the liquid. The housing 20 is stationary and the system 10 has a support structure 60 that supports the housing 20. The housing 20 is electrically insulated from the support structure 60.

The system 10 has a rigid rotating element 68 composed of a rotating-element shaft 70 and an agitator 198 attached to the rotating-element shaft 70. The rotating-element shaft 70 and the agitator 198 are centred on and can rotate around a rotating-element axis 72. The complete rotating element 68 is of steel and electrically conductive. The agitator 198 is located in the inner space 46 and the rotating-element shaft 70 extends from within the housing 20 to outside the housing 20, which means that the rotating element 68 is in part located in the inner space 46 and that the rotating-element shaft 70 is accessible from outside the housing 20. The system 10 has a rotating-element motor 74 that is coupled to rotating-element shaft 70 via a belt drive and can rotate the rotating element 68.

The system 10 has a first connector 32 and second connector 34 in the form of a ring lug connectors secured to the housing 20 by screws. The system 10 further has an electric first wire 36 connecting the first connector 32 to a first terminal 24 of the power source 22 and an electric second wire 38 connecting the second connector 34 to a second terminal 26 of the power source 22. In an alternative embodiment, the second terminal 26 is connected ground and the housing 20 is connected to ground via the support structure 60.

The first terminal 24 of the power source 22 can supply an alternating current with a square waveform. The current is about 0.5 mA, has a frequency below of 1 Hz, and is supplied at a peak voltage of about 50 V.

The system 10 further has a first internal connector 40 in the form of a metal rolling-element bearing and a second internal connector 42 in the form of a metal rolling-element bearing that are located axially between the first connector 32 and the second connector 34 relative to the rotating-element axis 72. Both the first internal connector 40 and the second internal connector 42 electrically couple the rotating-element shaft 70 to the housing 20.

The rotating element 68 and the housing 20 are thus electrically coupled in an electric network 30. The agitator 198 is connected to the rotating element shaft 70 between the first internal connector 40 and the second internal connector 42, the rotating-element shaft 70 is be composed of a single shaft section, and the agitator 198 is coupled in parallel with the rotating element shaft 70 in the electric network 30. The electric network 30 forms part of an electric circuit 28 together with the power source 22, first wire 36, and second wire 38. The electric network 30 can distribute the current between the housing 20 and the rotating element 68, and the electric circuit 28 is arranged to lead the current through both the housing 28 and the rotating element 68. This way, the supplied current can contribute to reduce microbiological growth in the mixer 196.

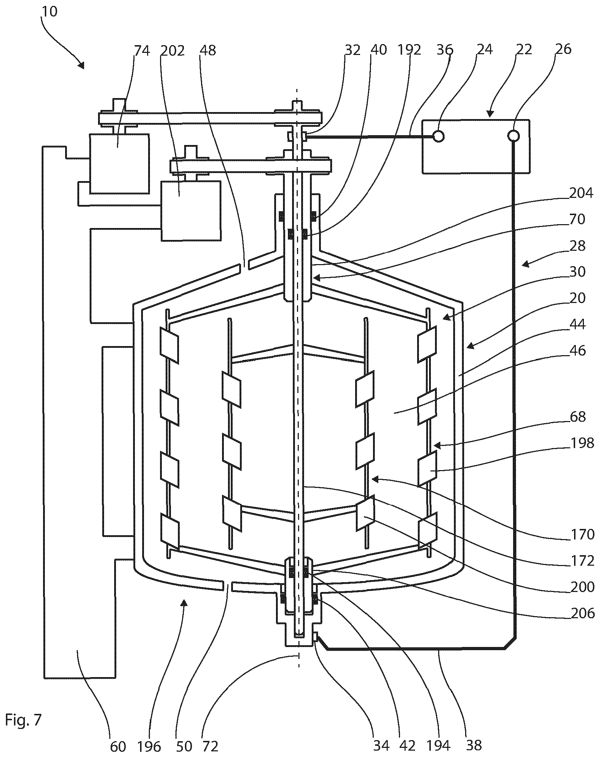

**Fig. 7** shows a system 10 with an electric power source 22 and a mixer 196. The system 10 differs from the system 10 described in relation to Fig. 6 in that it further has a rigid additional rotating element 170 composed of an additional rotating-element shaft 172 and an additional agitator 200 attached to the additional rotating-element shaft 172. The additional rotating-element shaft 172 is coaxial with the rotating-element shaft 70, and the additional rotating-element shaft 172 and the additional agitator 200 are centred on and can rotate around the rotating-element axis 72. The complete additional rotating element 170 is of steel and electrically conductive. The additional agitator 200 is located in the inner space 46 and the additional rotating-element shaft 172 extends from within the housing 20 to outside the housing 20, which means that the additional rotating element 170 is in part located in the inner space 46 and that the additional rotating-element shaft 172 is accessible from outside the housing 20. The system 10 has an additional rotating-element motor 202 that is coupled to the additional rotating-element shaft 172 via a belt drive and can rotate the additional rotating element 170. This way, the rotating element shaft 70 and the additional rotating-element shaft 172 can rotate at different rates and in different directions.

The system 10 further differs from that of Fig. 6 in that the first connector 32 is a slip ring that connects to the additional rotating-element shaft 172.

The system 10 further has a third internal connector 192 and a fourth internal connector 194 in the form of a metal rolling-element bearings that are located axially between the first connector 32 and the second connector 34 relative to the rotating-element axis 72. Both the third internal connector 192 and the fourth internal connector 194 electrically couples the additional rotating-element shaft 172 to the rotating element shaft 70. In an alternative embodiment, the fourth internal connector 194 electrically couples the additional rotating-element shaft 172 to the housing 20. The rotating element 68, the additional rotating element 170, and the housing 20 are thus electrically coupled in the electric network 30.

The agitator 198 is connected to the rotating element shaft 70 between the first internal connector 40 and the second internal connector 42. The rotating-element shaft 70 is composed of a first shaft section 204 and a second shaft section 206 that are interconnected by the agitator 198, and the first shaft section 204, the agitator 198, and the second shaft section 206 are coupled in series. The electric network 30 forms part of the electric circuit 28 together with the power source 22, first wire 36, and second wire 38. The electric network 30 can distribute the current between the housing 20, the rotating element 68, and the additional rotating element 170, and the electric circuit 28 is arranged to lead the current through the housing 28, the rotating element 68, and the additional rotating element 70. This way, the supplied current can contribute to reduce microbiological growth in the mixer 196.

### Item list

10 system
12 centrifugal pump
14 extruder
20 housing
22 electric power source
24 first terminal
26 second terminal
28 electric circuit
30 electric network
32 first connector
34 second connector
36 electric first wire
38 electric second wire
40 first internal connector
42 second internal connector-
44 wall
46 inner space
48 inlet
50 outlet
60 support structure
64 feed pipe
66 discharge pipe
68 rotating element
70 rotating-element shaft
72 rotating-element axis
74 rotating-element motor
84 impeller
86 rear hub plate
88 forward hub plate
90 vanes
92 impeller eye
94 rotor
96 nacelle
166 rotary-type positive-displacement pump
168 rotor
170 additional rotating element
172 additional rotating-element shaft
174 additional rotor
176 additional rotating-element axis
178 gear train
180 gear
182 additional gear
184 hub
186 lobe
188 additional hub
190 additional lobe
192 third internal connector
194 fourth internal connector
196 mixer
198 agitator
200 additional agitator
202 additional rotating-element motor
204 first shaft section
206 second shaft section

## Claims

1. A system (10) comprising:
- a housing (20),
- an electric power source (22), and
- a rotating element (68), wherein
the housing (20) is electrically conductive and arranged to hold a fluid, the power source (22) is arranged to supply an electric current, the housing (20) and the electric power source (22) are operationally coupled in an electric circuit (28) arranged to lead the current from the electric power source (22) through the housing (20), and the electric current is configured to inhibit microbiological growth within the housing (20),
**characterized in that** the system (10) further comprises:
- a rotating element (68), wherein
the housing (20) is a static housing (20), the rotating element (68) is located at least partly within the housing (20), the rotating element (68) is arranged to rotate around a rotating-element axis (72), and the rotating element (68) and the housing (20) are electrically coupled in an electric network (30) that forms part of the electric circuit (28).

2. The system (10) according to claim 1, wherein the system (10) further comprises:
- a first internal connector (40) that electrically couples the rotating element (68) to the housing (20), and the first internal connector (40) is a rotary electrical connector.

3. The system (10) according to claim 1 or 2, wherein the housing (20) and the rotating element (68) form part of a centrifugal pump (12).

4. The system (10) according to claim 3, wherein the rotating element (68) comprises:
- a rotating-element shaft (70), and
- an impeller (84), wherein
the housing (20) has an inlet (48) for receiving the fluid into the housing (20) and an outlet (50) for discharging the fluid from the housing (20), the rotating-element shaft (70) extends from within the housing (20) to outside the housing (20) and is arranged to rotate around the rotating-element axis (72) relative to the housing (20), and the impeller (84) is attached to the rotating-element shaft (70) and arranged to generate a flow of the fluid from the inlet (48) to the outlet (50) at a rotation around the rotating-element axis (72).

5. The system (10) according to claim 1 or 2, wherein the housing (20) and the rotating element (68) form part of an extruder.

6. The system (10) according to claim 6, wherein the rotating element (68) comprises:
- a rotating-element shaft (70), and
- a rotor (94), wherein
the housing (20) has an inlet (48) for receiving the fluid into the housing (20) and an outlet (50) for discharging the fluid from the housing (20), the rotating-element shaft (70) extends from within the housing (20) to outside the housing (20) and is arranged to rotate around the rotating-element axis (72) relative to the housing (20), and the rotor (94) is attached to the rotating-element shaft (70) and arranged to generate a flow of the fluid from the inlet (48) to the outlet (50) at a rotation around the rotating-element axis (72).

7. The system (10) according to any of the claims 1 to 6, wherein the electric circuit (28) is connected to ground.

8. The system (10) according to any of the claims 1 to 7, wherein the current is an alternating current, the current has a square waveform, the current is below 10 mA, and the current is greater than 0.01 mA.

## Patentansprüche

1. System (10), umfassend:
- ein Gehäuse (20),
- eine elektrische Energiequelle (22), und
- ein rotierendes Element (68), wobei
das Gehäuse (20) elektrisch leitend und so angeordnet ist, dass es ein Fluid hält, die Energiequelle (22) so angeordnet ist, dass sie einen elektrischen Strom liefert, das Gehäuse (20) und die elektrische Energiequelle (22) betriebsmäßig in einem elektrischen Stromkreis (28) gekoppelt sind, der so angeordnet ist, dass er den Strom von der elektrischen Energiequelle (22) durch das Gehäuse (20) leitet, und der elektrische Strom so konfiguriert ist, dass er mikrobiologisches Wachstum innerhalb des Gehäuses (20) hemmt,
**dadurch gekennzeichnet, dass** das System (10) ferner umfasst:
- ein rotierendes Element (68), wobei
das Gehäuse (20) ein statisches Gehäuse (20) ist, das rotierende Element (68) sich wenigstens teilweise innerhalb des Gehäuses (20) befindet, das rotierende Element (68) so angeordnet ist, dass es um eine Achse (72) des rotierenden Elements rotiert, und das rotierende Element (68) und das Gehäuse (20) in einem elektrischen Netzwerk (30), das Teil des elektrischen Stromkreises (28) ist, elektrisch gekoppelt sind.

2. System (10) nach Anspruch 1, wobei das System (10) ferner umfasst:
- einen ersten internen Verbinder (40), der das rotierende Element (68) elektrisch mit dem Gehäuse (20) koppelt, und der erste interne Verbinder (40) ein elektrischer Drehverbinder ist.

3. System (10) nach Anspruch 1 oder 2, wobei das Gehäuse (20) und das rotierende Element (68) Teil einer Zentrifugalpumpe (12) sind.

4. System (10) nach Anspruch 3, wobei das rotierende Element (68) umfasst:
- eine Welle (70) des rotierenden Elements, und
- ein Laufrad (84), wobei
das Gehäuse (20) einen Einlass (48) zur Aufnahme des Fluids in das Gehäuse (20) und einen Auslass (50) zum Abführen des Fluids aus dem Gehäuse (20) aufweist, die Welle (70) des rotierenden Elements sich von innerhalb des Gehäuses (20) nach außerhalb des Gehäuses (20) erstreckt und so angeordnet ist, dass sie sich um die Achse (72) des rotierenden Elements relativ zum Gehäuse (20) dreht, und das Laufrad (84) an der Welle (70) des rotierenden Elements befestigt und so angeordnet ist, dass es bei einer Drehung um die Achse (72) des rotierenden Elements einen Strom des Fluids vom Einlass (48) zum Auslass (50) erzeugt.

5. System (10) nach Anspruch 1 oder 2, wobei das Gehäuse (20) und das rotierende Element (68) Teil eines Extruders sind.

6. System (10) nach Anspruch 6, wobei das rotierende Element (68) umfasst:
- eine Welle (70) des rotierenden Elements, und
- einen Rotor (94), wobei
das Gehäuse (20) einen Einlass (48) zur Aufnahme des Fluids in das Gehäuse (20) und einen Auslass (50) zum Abführen des Fluids aus dem Gehäuse (20) aufweist, die Welle (70) des rotierenden Elements sich von innerhalb des Gehäuses (20) nach außerhalb des Gehäuses (20) erstreckt und so angeordnet ist, dass sie sich um die Achse (72) des rotierenden Elements relativ zum Gehäuse (20) dreht, und der Rotor (94) an der Welle (70) des rotierenden Elements befestigt und so angeordnet ist, dass es bei einer Drehung um die Achse (72) des rotierenden Elements einen Strom des Fluids vom Einlass (48) zum Auslass (50) erzeugt.

7. System (10) nach einem der Ansprüche 1 bis 6, wobei der elektrische Stromkreis (28) mit Masse verbunden ist.

8. System (10) nach einem der Ansprüche 1 bis 7, wobei der Strom ein Wechselstrom ist, der Strom eine Rechteckwellenform aufweist, der Strom unter 10 mA liegt und der Strom größer als 0,01 mA ist.

## Revendications

1. Système (10) comprenant :
- un boîtier (20),
- une source d'alimentation électrique (22), et
- un élément rotatif (68), dans lequel
le boîtier (20) est électriquement conducteur et agencé pour contenir un fluide, la source d'alimentation (22) est agencée pour fournir un courant électrique, le boîtier (20) et la source d'alimentation électrique (22) sont couplés de manière opérationnelle dans un circuit électrique (28) agencé pour conduire le courant de la source d'alimentation électrique (22) à travers le boîtier (20), et le courant électrique est configuré pour inhiber la croissance microbiologique à l'intérieur du boîtier (20),
**caractérisé en ce que** le système (10) comprend également :
- un élément rotatif (68), dans lequel
le boîtier (20) est un boîtier statique (20), l'élément rotatif (68) est situé au moins en partie à l'intérieur du boîtier (20), l'élément rotatif (68) est agencé pour tourner autour d'un axe d'élément rotatif (72), et l'élément rotatif (68) et le boîtier (20) sont couplés électriquement dans un réseau électrique (30) qui fait partie du circuit électrique (28).

2. Système (10) selon la revendication 1, dans lequel le système (10) comprend également :
- un premier connecteur interne (40) qui couple électriquement l'élément rotatif (68) au boîtier (20), et le premier connecteur interne (40) est un connecteur électrique rotatif.

3. Système (10) selon la revendication 1 ou 2, dans lequel le boîtier (20) et l'élément rotatif (68) font partie d'une pompe centrifuge (12).

4. Système (10) selon la revendication 3, dans lequel l'élément rotatif (68) comprend :
- un arbre d'élément rotatif (70), et
- une roue à aubes (84), dans lequel
le boîtier (20) présente une entrée (48) destinée à recevoir le fluide dans le boîtier (20) et une sortie (50) destinée à évacuer le fluide du boîtier (20), l'arbre d'élément rotatif (70) se prolonge de l'intérieur du boîtier (20) vers l'extérieur du boîtier (20) et est agencé pour tourner autour de l'axe d'élément rotatif (72) par rapport au boîtier (20), et la roue à aubes (84) est fixée à l'arbre d'élément rotatif (70) et agencée pour générer un écoulement du fluide de l'entrée (48) vers la sortie (50) lors d'une rotation autour de l'axe d'élément rotatif (72).

5. Système (10) selon la revendication 1 ou 2, dans lequel le boîtier (20) et l'élément rotatif (68) font partie d'une extrudeuse.

6. Système (10) selon la revendication 6, dans lequel l'élément rotatif (68) comprend :
- un arbre d'élément rotatif (70), et
- un rotor (94), dans lequel
le boîtier (20) présente une entrée (48) destinée à recevoir le fluide dans le boîtier (20) et une sortie (50) destinée à évacuer le fluide du boîtier (20), l'arbre d'élément rotatif (70) se prolonge de l'intérieur du boîtier (20) vers l'extérieur du boîtier (20) et est agencé pour tourner autour de l'axe d'élément rotatif (72) par rapport au boîtier (20), et le rotor (94) est fixé à l'arbre d'élément rotatif (70) et agencé pour générer un écoulement du fluide de l'entrée (48) vers la sortie (50) lors d'une rotation autour de l'axe d'élément rotatif (72).

7. Système (10) selon l'une quelconque des revendications 1 à 6, dans lequel le circuit électrique (28) est connecté à la masse.

8. Système (10) selon l'une quelconque des revendications 1 à 7, dans lequel le courant est un courant alternatif, le courant a une forme d'onde carrée, le courant est inférieur à 10 mA et le courant est supérieur à 0,01 mA.
